# EUROPEAN PATENT APPLICATION

(11) **EP 4 049 724 A1**
(43) Date of publication of application: **31.08.2022**
(21) Application number: 20879043.6
(22) Date of filing: 23.10.2020
(51) Int. Cl.: A61P 19/02, A61P 19/04, A61P 43/00, A61K 31/655

(54) **PHARMACEUTICAL COMPOSITION FOR PROTECTING CARTILAGE**

(30) Priority: 24.10.2019 JP 2019193329
(71) Applicant: Kyoto University, Kyoto-shi, Kyoto 606-8501 (JP); Kyoto Drug Discovery & Development Co., Ltd., Ibaraki-shi, Osaka, 567-0085 (JP)
(72) Inventor: NISHITANI, Kohei, Kyoto-shi, Kyoto 606-8501 (JP); KAKIZUKA, Akira, Kyoto-shi, Kyoto 606-8501 (JP); IKEDA, Hanako, Kyoto-shi, Kyoto 606-8501 (JP); MATSUDA, Shuichi, Kyoto-shi, Kyoto 606-8501 (JP); IWAI, Sachiko, Kyoto-shi, Kyoto 606-8501 (JP); SAITO, Motoo, Kyoto-shi, Kyoto 606-8501 (JP); MUSASHI, Kunihiro, Ibaraki-shi, Osaka 567-0085 (JP)
(74) Representative: Hoffmann Eitle
(86) International application number: PCT/JP2020/039896
(87) International publication number: WO 2021/079983

(57) **Abstract**

The inventors have found that compounds of formula (I) have an effect to protect cartilage. Accordingly the disclosure provides a pharmaceutical composition for protecting cartilage comprising a compound of formula (I) or an ester, oxide, pharmaceutically acceptable salt, or solvate thereof. The pharmaceutical composition can be used for treating and/or preventing a disease associated with cartilage degeneration such as osteoarthritis.

## Description

### TECHNICAL FIELD

This application claims the benefit of priority of Japanese Patent Application No. 2019-193329, the entire contents of which are incorporated herein by reference.

The disclosure relates to a pharmaceutical composition for protecting cartilage.

### BACKGROUND

Articular cartilage is hyaline cartilage and is composed of chondrocytes and cartilage matrix. Cartilage matrix is produced by chondrocytes and composed of type 2 collagen and proteoglycans such as chondroitin sulfate. Hyaline cartilage is an avascular tissue and hard to heal. Once hyaline cartilage is damaged, it is not repaired as hyaline cartilage and turns to fibrocartilage.

Osteoarthritis is a major pathology of articular cartilage. The number of patients in Japan is estimated to be over 20 million and the number of patients who have symptom with impaired QOL such as pain, difficulty in walking, or difficulty in daily life is estimated to be 7 to 10 million. Cartilage damage after a fracture or trauma also impairs QOL and then progresses to secondary osteoarthritis. Such damages to articular cartilage are irreversible. At present no medicine is available for repairing cartilage, and only symptomatic treatment is available. When osteoarthritis progresses to its terminal stage and QOL is significantly impaired, joint replacement surgery is performed to remove all damaged articular cartilage and replace the entire joint with an artificial joint. The number of joint replacement surgery is increasing worldwide and significant medical expenses are incurred.

Attempts to repair articular cartilage with a medicine have been reported. For example, protection of articular cartilage by inhibiting Wnt-β-catenin was examined in a clinical trial (Non-Patent Literature 1). An unsaturated fatty acid was reported to have an effect to inhibit apoptosis of chondrocytes and protect chondrocytes (Non-Patent Literature 2). However, no medicine has been available for practical use.

Certain 4-amino-naphthalene-1-sulfonic acid derivatives have a VCP (valosin-containing protein) ATPase inhibitory activity and are considered to be effective for treating various diseases (Patent Literature 1). Especially, they are known to be effective in treatment and/or prevention of some ocular diseases and leptin resistance (Patent Literatures 2 to 6).

### REFERENCES

### PATENT LITERATURE

[Patent Literature 1] WO2012/014994
[Patent Literature 2] WO2012/043891
[Patent Literature 3] WO2014/129495
[Patent Literature 4] WO2015/129809
[Patent Literature 5] WO2015/033981
[Patent Literature 6] WO2019/131720

### NON-PATENT LITERATURE

[Non-Patent Literature 1] Yazici Y, et al., Osteoarthritis Cartilage. 2017 Oct;25(10):1598-1606.
[Non-Patent Literature 2] Sakata S, et al., J Orthop Res. 2015 Mar;33(3):359-65.

### SUMMARY

An object of the disclosure is to provide a pharmaceutical composition for protecting cartilage.

The inventors have found that VCP inhibitors have an effect to protect cartilage and are effective for treating and/or preventing a disease associated with cartilage degeneration.

Accordingly, an aspect of the disclosure provides a pharmaceutical composition for protecting cartilage comprising a compound of formula (I): 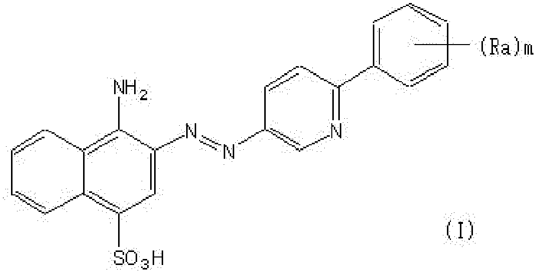 wherein
Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
m is an integer selected from 0 to 4,
or an ester, oxide, pharmaceutically acceptable salt, or solvate thereof.

Another aspect of the disclosure provides a pharmaceutical composition for treating and/or preventing a disease associated with cartilage degeneration comprising a compound of formula (I) or an ester, oxide, pharmaceutically acceptable salt, or solvate thereof.

An effect of the disclosure is the provision of the pharmaceutical composition for protecting cartilage.

### BRIEF DESCRIPTION OF DRAWINGS

Fig. 1 shows relative number of viable cells in mouse chondrocytes cultured in the presence of tunicamycin.
Fig. 2 shows relative number of viable cells in mouse chondrocytes cultured under glucose starvation stress.
Fig. 3 shows relative number of viable cells in mouse chondrocytes cultured in the presence of TNFα.
Fig. 4 shows results of Western blotting for measuring Bip and CHOP in mouse chondrocytes cultured in the presence of tunicamycin.
Fig. 5 shows results of Western blotting for measuring cCap3 in mouse chondrocytes cultured in the presence of TNFα.
Fig. 6 shows relative number of viable cells in human chondrocytes cultured in the presence of tunicamycin.
Fig. 7 shows caspase 3/7 activity of human chondrocytes cultured in the presence of tunicamycin.
Fig. 8 shows results of Western blotting for measuring Bip, ATF4, and CHOP in human chondrocytes cultured in the presence of tunicamycin. The expression levels of ATF4 and CHOP in the absence of tunicamycin are 0.001 ± 0.001 and 0.01 ± 0.02, respectively.
Fig. 9 shows results of Western blotting for measuring p-JNK and JNK in human chondrocytes cultured in the presence of tunicamycin.
Fig. 10 shows gene expression levels of MMP13 and MMP1 in human chondrocytes cultured in the presence of IL-1β, which were determined by real-time PCR.
Fig. 11 shows relative number of viable cells in human femorotibial joint cartilage cultured in organ culture.
Fig. 12 shows images of sections which were prepared from knee cartilages of rats treated with monoiodoacetate (MIA) and were stained with Safranin-O.
Fig. 13 shows magnified images of the tibial joints shown in Fig. 12.
Fig. 14 shows ORSI scores of rats treated with MIA. Mean values of the scores of medial femur, lateral femur, medial tibia, and lateral tibia are shown.
Fig. 15 shows Mankin scores of posterolateral femoral condyles of traumatic cartilage damage model rats. The scores were evaluated two or four weeks after periodic compression to knee joints.
Fig. 16 shows volumes of cartilage damage in posterolateral femoral condyles of traumatic cartilage damage model rats. The volumes were determined four weeks after periodic compression to knee joints.
Fig. 17 shows numbers of chondrocytes in posterolateral femoral condyles of traumatic cartilage damage model rats. The numbers were determined four weeks after periodic compression to knee joints.

### DETAILED DESCRIPTION

When a numerical value is accompanied with the term "about", the value is intended to represent any value in the range of -10% of the value to +10% of the value. For example, "about 20" means "a value from 18 to 22." A range defined with values of the lower and upper limits covers all values from the lower limit to the upper limit, including the values of the both limits. When a range is accompanied with the term "about", the both limits are read as accompanied with the term. For example, "about 20 to 30" is read as "18 to 33."

Unless otherwise defined, the terms used herein are read as generally understood by those skilled in the technical fields such as organic chemistry, medical sciences, pharmaceutical sciences, molecular biology, and microbiology. Several terms used herein are defined as below. The definitions herein take precedence over the general understanding.

The term "alkyl" refers to a monovalent saturated aliphatic hydrocarbyl group having 1 to 10, preferably 1 to 6, carbon atoms. Examples of the alkyl groups include, but are not limited to, linear and branched hydrocarbyl groups, such as methyl, ethyl, n-propyl, isopropyl, n-butyl, isobutyl, sec-butyl, t-butyl, n-pentyl, and neopentyl.

The term "substituted" as a word qualifying a name of a group indicates that one or more hydrogen atoms of the group are, identically or differently, replaced with one or more designated substituents.

The term "alkylene" refers to a divalent saturated aliphatic hydrocarbyl group having 1 to 10, preferably 1 to 6, carbon atoms. The alkylene groups include branched and straight chain hydrocarbyl groups.

The term "alkoxy" refers to an -O-alkyl group in which the alkyl group is as defined herein. Examples of the alkoxy groups include, but are not limited to, methoxy, ethoxy, n-propoxy, isopropoxy, n-butoxy, t-butoxy, sec-butoxy, and n-pentoxy.

The term "aryl" refers to a monovalent aromatic carbocyclic group of 6 to 14 carbon atoms having a single ring (e.g., phenyl) or multiple condensed rings (e.g., naphthyl or anthryl). The aryl groups typically include phenyl and naphthyl.

The term "aryloxy" refers to an -O-aryl group in which the aryl group is as defined herein, including, e.g., phenoxy and naphthoxy.

The term "cyano" refers to the -CN group.

The term "carboxyl" or "carboxy" refers to the -COOH group or a salt thereof.

The term "carboxy ester" refers to a -C(O)O-alkyl group in which the alkyl group is as defined herein.

The term "halo" refers to a halogen, especially fluoro, chloro, bromo, or iodo.

The term "hydroxy" refers to the -OH group.

A substituent that is not explicitly defined herein is named by describing the name of the terminal functional group of the substituent first and sequentially describing the adjacent functional group(s) toward the binding point of the substituent, unless otherwise indicated. For example, the substituent "arylalkyloxycarbonyl" refers to (aryl)-(alkyl)-O-C(O)-.

Some compounds of formula (I) have enantiomers or diastereomers, depending on arrangements of their substituents. Some compounds of formula (I) may be provided as racemic mixtures or may be provided in stereoisomerically pure forms separated by a known method. Some compounds of formula (I) may be tautomers.

The term "ester" refers to an ester that hydrolyzes in vivo, which may break down readily in a human body to leave the parent compound or a salt thereof. Suitable esters include, for example, those derived from pharmaceutically acceptable aliphatic carboxylic acids, especially alkanoic, alkenoic, cycloalkanoic, and alkanedioic acids, in which each alkyl or alkenyl group has, for example, not more than six carbon atoms. Examples of the esters include formates, acetates, propionates, butyrates, acrylates, and ethylsuccinates.

The term "oxide" refers to an oxide in which a nitrogen in a heteroaryl group is oxidized to form N-oxide.

The term "pharmaceutically acceptable salt" may indicate a salt of a compound of formula (I) with an inorganic or organic acid. Preferred salts include salts with inorganic acids, such as hydrochloric acid, hydrobromic acid, phosphoric acid, and sulfuric acid, and salts with organic carboxylic acids and sulfonic acids, such as acetic acid, trifluoroacetic acid, propionic acid, maleic acid, fumaric acid, malic acid, citric acid, tartaric acid, lactic acid, benzoic acid, methanesulfonic acid, ethanesulfonic acid, benzenesulfonic acid, toluenesulfonic acid, naphthalene sulfonic acid, and naphthalene disulfonic acid.

Pharmaceutically acceptable salts also include salts with conventional bases, such as alkali metal salts, e.g., a sodium salt and a potassium salt, alkaline earth metal salts, e.g., a calcium salt and a magnesium salt, ammonium salts derived from ammonia and organic amines, e.g., diethylamine, triethylamine, ethyldiisopropylamine, procaine, dibenzylamine, N-methylmorpholine, dihydroabiethylamine, methylpiperidine, L-arginine, creatine, choline, L-lysine, ethylenediamine, benzathine, ethanolamine, meglumine, and tromethamine, especially a sodium salt.

The term "solvate" means a compound of formula (I) which is coordinated with a solvent molecule to form a complex in a solid or liquid state. A suitable solvate is a hydrate.

The term "compound of formula (I)" as used herein is intended to include its esters, oxides, pharmaceutically acceptable salts, and solvates, as long as the context allows it.

In an embodiment, each Ra radical in formula (I) is independently selected from the group consisting of halo, hydroxy, alkyl, halo-substituted alkyl, and alkoxy.

In an embodiment, each Ra radical in formula (I) is independently selected from the group consisting of halo and alkyl.

In an embodiment, formula (I) has two Ra radicals which are halo and alkyl.

In an embodiment, the compound of formula (I) is selected from the compounds listed in Table 1 below:

**[Table 1-1]**

| No. | Compound Name |
|---|---|
| 1 | 4-amino-3-(6-phenylpyridine-3-ylazo)naphthalene-1-sulfonic acid |
| 2 | 4-amino-3-(6-p-tolylpyridine-3-ylazo)naphthalene-1-sulfoni c acid |
| 3 | 4-amino-3-(6-m-tolylpyridine-3-ylazo)naphthalene-1-sulfoni c acid |
| 4 | 4-amino-3-(6-o-tolylpyridine-3-ylazo)naphthalene-1-sulfoni c acid |
| 5 | 4-amino-3-(6-biphenyl-2-ylpyridine-3-ylazo)naphthalene-1-s ulfonic acid |
| 6 | 3-[6-(2-acetylphenyl)pyridine-3-ylazo]-4-aminonaphthalene-1-sulfonic acid |
| 7 | 3-[6-(3-acetylphenyl)pyridine-3-ylazo]-4-aminonaphthalene-1-sulfonic acid |
| 8 | 3-[6-(4-acetylphenyl)pyridine-3-ylazo]-4-aminonaphthalenes ulfonic acid |
| 9 | 4-amino-3-[6-(2,4-dichlorophenyl)pyridine-3-ylazo]naphthal ene-1-sulfonic acid |
| 10 | 4-amino-3-[6-(2-trifluoromethylphenyl)pyridine-3-ylazo]nap hthalene-1-sulfonic acid |
| 11 | 4-amino-3-[6-(4-trifluoromethylphenyl)pyridine-3-ylazo]nap hthalene-1-sulfonic acid |
| 12 | 4-amino-3-[6-(2-chlorophenyl)pyridine-3-ylazo]naphthalene-1-sulfonic acid |
| 13 | 4-amino-3-[6-(3-chlorophenyl)pyridine-3-ylazo]naphthalene-1-sulfonic acid |
| 14 | 4-amino-3-[6-(4-chlorophenyl)pyridine-3-ylazo]naphthalene-1-sulfonic acid |
| 15 | 4-amino-3-[6-(2-methoxyphenyl)pyridine-3-ylazo]naphthalene -1-sulfonic acid |
| 16 | 4-amino-3-[6-(4-methoxyphenyl)pyridine-3-ylazo]naphthalene -1-sulfonic acid |
| 17 | 4-amino-3-[6-(2-isopropoxyphenyl)pyridine-3-ylazo]naphthal ene-1-sulfonic acid |
| 18 | 4-amino-3-[6-(4-isopropoxyphenyl)pyridine-3-ylazo]naphthalene-1-sulfonic acid |
| 19 | 4-amino-3-[6-(2-phenoxyphenyl)pyridine-3-ylazo]naphthalene -1-sulfonic acid |
| 20 | 4-amino-3-[6-(3-methoxyphenyl)pyridine-3-ylazo]naphthalene -1-sulfonic acid |

**[Table 1-2]**

| | |
|---|---|
| 21 | 4-amino-3-[6-(2,3-dimethylphenyl)pyridine-3-ylazo]naphthal ene-1-sulfonic acid |
| 22 | 4-amino-3-[6-(2,5-dimethylphenyl)pyridine-3-ylazo]naphthal ene-1-sulfonic acid |
| 23 | 4-amino-3-[6-(3,5-dimethylphenyl)pyridine-3-ylazo]naphthal ene-1-sulfonic acid |
| 24 | 4-amino-3-[6-(3-trifluoromethylphenyl)pyridine-3-ylazo]nap hthalene-1-sulfonic acid |
| 25 | 4-{4-[5-(1-amino-4-sulfonaphthalene-2-ylazo)pyridine-2-yl ]phenyl}-4-oxobutyric acid |
| 26 | 4-amino-3-(6-biphenyl-3-ylpyridine-3-ylazo)naphthalene-1-s ulfonic acid |
| 27 | 4-amino-3-[6-(3-cyanophenyl)pyridine-3-ylazo]naphthalene-1 -sulfonic acid |
| 28 | 4-amino-3-[6-(4-cyanophenyl)pyridine-3-ylazo]naphthalene-1 -sulfonic acid |
| 29 | 4-amino-3-[6-(3,5-bistrifluoromethylphenyl)pyridine-3-ylaz o]naphthalenesulfonic acid |
| 30 | 4-amino-3-[6-(4-benzoylphenyl)pyridine-3-ylazo]naphthalene -1-sulfonic acid |
| 31 | 4-amino-3-[6-(2-propoxyphenyl)pyridine-3-ylazo]naphthalen e-1-sulfonic acid |
| 32 | 4-amino-3-[6-(4-fluoro-2-methylphenyl)pyridine-3-ylazo]nap hthalene-1-sulfonic acid |
| 33 | 4-amino-3-[6-(5-fluoro-2-propoxyphenyl)pyridine-3-ylazo]na phthalene-1-sulfonic acid |
| 34 | 4-amino-3-[6-(2-fluoro-6-propoxyphenyl)pyridine-3-ylazo]na phthalene-1-sulfonic acid |
| 35 | 4-amino-3-[6-(4-fluoro-2-propoxyphenyl)pyridine-3-ylazo]na phthalene-1-sulfonic acid |
| 36 | 4-amino-3-[6-(5-fluoro-2-methylphenyl)pyridine-3-ylazo]nap hthalene-1-sulfonic acid |
| 37 | 4-amino-3-[6-(2-fluoro-5-methylphenyl)pyridine-3-ylazo]nap hthalene-1-sulfonic acid |
| 38 | 4-amino-3-[6-(2-butoxyphenyl)pyridine-3-ylazo]naphthalene-1-sulfonic acid |
| 39 | 4-amino-3-[6-(2-hexyloxyphenyl)pyridine-3-ylazo]naphthalen e-1-sulfonic acid |
| 40 | 4-amino-3-[6-(4-butylphenyl)pyridine-3-ylazo]naphthalene-1 -sulfonic acid |

**[Table 1-3]**

| | |
|---|---|
| 41 | 4-amino-3-[6-(2-hydroxyphenyl)pyridine-3-ylazo]naphthalene -1-sulfonic acid |
| 42 | 4-amino-3-{6-[2-(6-hydroxyhexyloxy)phenyl]pyridine-3-ylazo }naphthalene-1-sulfonic acid |
| 43 | 4-{2-[5-(1-amino-4-sulfonaphthalene-2-ylazo)pyridine-2-yl] phenoxy}butyric acid |
| 44 | 4-amino-3-{6-[2-(3-hydroxypropoxy)phenyl]pyridine-3-ylazo} naphthalene-1-sulfonic acid |
| 45 | 4-amino-3-[6-(2-isobutoxyphenyl)pyridine-3-ylazo]naphthale ne-1-sulfonic acid |
| 46 | 4-amino-3-[6-(5-chloro-2-hydroxyphenyl)pyridine-3-ylazo]na phthalene-1-sulfonic acid |
| 47 | 4-amino-3-[6-(4-methylbiphenyl-2-yl)pyridine-3-ylazo]napht halene-1-sulfonic acid |
| 48 | 4-amino-3-[6-(4'-chloro-4-methylbiphenyl-2-yl)pyridine-3-y lazo]naphthalene-1-sulfonic acid |
| 49 | 4-amino-3-[6-(4,3',5'-trimethylbiphenyl-2-yl)pyridine-3-yl azo]naphthalene-1-sulfonic acid |
| 50 | 4-amino-3-[6-(3'-chloro-4-methylbiphenyl-2-yl)pyridine-3-y lazo]naphthalene-1-sulfonic acid |
| 51 | 4-amino-3-[6-(2,6-dimethylphenyl)pyridine-3-ylazo]naphthal ene-1-sulfonic acid |
| 52 | 4-amino-3-[6-(3-formyl-2-isopropoxy-5-methylphenyl)pyridin e-3-ylazo]naphthalene-1-sulfonic acid |
| 53 | 4-amino-3-[6-(3-formyl-2-butoxy-5-methylphenyl)pyridine-3-ylazo]naphthalene-1-sulfonic acid |

In an embodiment, the active ingredient of the pharmaceutical composition is 4-amino-3-[6-(4-fluoro-2-methylphenyl)pyridine-3-ylazo]naphthalene-1-sulfonic acid, which is represented by the following formula: 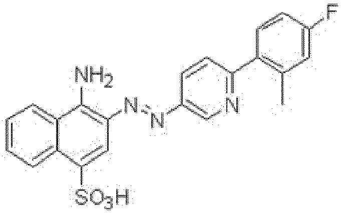 , or an ester, oxide, pharmaceutically acceptable salt, or solvate thereof, preferably the sodium salt.

The characteristics of the compounds of formula (I), especially the compounds listed above, and the methods for synthesizing them are described in WO2012/014994 (Patent Literature 1) in detail.

As demonstrated by the examples described below, the compounds of formula (I) can protect cartilage. Accordingly, an aspect of the disclosure provides a pharmaceutical composition for protecting cartilage comprising a compound of formula (I). As used herein, "protecting cartilage" or "protection of cartilage" includes protecting a chondrocyte, inhibiting expression of a cartilage-destroying enzyme (e.g., a cartilage matrix-degrading enzyme such as a matrix metalloproteinase), and/or inhibiting degeneration of cartilage matrix. Protecting a chondrocyte includes inhibiting death of a chondrocyte. In an embodiment, protecting cartilage may maintain structure and/or function of cartilage, prevent cartilage degeneration, and/or suppress decrease of cartilage function. Thus, the pharmaceutical composition can protect cartilage, for example, when cartilage is damaged in an intra-articular fracture, a ligament injury, or joint surgery. The pharmaceutical composition may also be used to protect cartilage in treating and/or preventing cartilage degeneration, or in treating and/or preventing a disease associated with cartilage degeneration, such as osteoarthritis.

The examples described below further demonstrate that the compounds of formula (I) inhibit cartilage degeneration in a traumatic cartilage damage model. Accordingly, an aspect of the disclosure provides a pharmaceutical composition for treating and/or preventing cartilage degeneration. An aspect of the disclosure provides a pharmaceutical composition for treating and/or preventing a disease associated with cartilage degeneration. Examples of the diseases associated with cartilage degeneration include osteoarthritis. Osteoarthritis includes primary osteoarthritis and secondary osteoarthritis, and especially post-traumatic secondary osteoarthritis. Osteoarthritis may occur in any joint and includes, for example, temporomandibular joint osteoarthritis, knee osteoarthritis, hip osteoarthritis, ankle osteoarthritis, shoulder osteoarthritis, elbow osteoarthritis, wrist osteoarthritis, finger osteoarthritis, or spondylosis.

The term "treating" or "treatment" as used herein means that in a subject suffering from a disease a cause of the disease is reduced or removed, progression of the disease is delayed or stopped, and/or a symptom of the disease is reduced, alleviated, ameliorated, or removed.

The term "preventing" or "prevention" as used herein means that in a subject, especially a subject that is susceptible to a disease but has not been affected with the disease yet, the disease onset is prevented or the possibility of the disease onset is decreased. Examples of the subjects that are susceptible to osteoarthritis but have not been affected with it yet include subjects having a risk factor of osteoarthritis. Examples of the risk factors include, but are not limited to, heredity, occupation, smoking, metabolic diseases (e.g., hyperlipidemia, hypertension, obesity), cartilage fragility, subchondral bone fragility, osteoporosis, trauma, joint dysplasia, flail joint, and advanced age (e.g., persons over 50 years, especially over 60 years).

The subjects to be treated with the pharmaceutical composition include animals, typically mammals (e.g., humans, mice, rats, hamsters, rabbits, cats, dogs, cows, sheep, or monkeys), especially humans.

The pharmaceutical composition may be administered in any way through a conventional administration route, for example, by oral administration, parenteral administration, injection, or infusion. The composition may be in a dosage form suitable for each administration route. In an embodiment, the pharmaceutical composition is administered intraarticularly. In an embodiment, the pharmaceutical composition is administered intravenously.

Dosage forms suitable for oral administration include granules, fine granules, powders, coated tablets, tablets, suppositories, fine powders, capsules, microcapsules, chewable tablets, liquids, suspensions, and emulsions. Dosage forms suitable for injection may be conventional pharmaceutical dosage forms, e.g., those suitable for intracoronary administration, intravenous injection, infusion, or formulations for extended release of active ingredients. Dosage forms for intraarticular administration, intravenous injection, or infusion include aqueous and non-aqueous injectable solutions, which may comprise excipients such as antioxidants, buffers, bacteriostatic agents, or isotonic agents; and aqueous and non-aqueous injectable suspensions, which may comprise excipients such as suspending agents or thickening agents. Such dosage forms may be provided as liquids in sealed ampoules or vials, or provided as lyophilized products to be prepared immediately prior to use by adding sterile liquids such as water for injection. The injectable solutions or suspensions may be prepared from powders, granules, or tablets.

Such dosage forms can be manufactured by a conventional formulation method. If necessary for the formulation, various pharmaceutically acceptable excipients may be added. Any excipient may be used in accordance with the employed dosage form. Examples of the excipients include buffering agents, surfactants, stabilizers, preservatives, fillers, diluents, additives, disintegrants, binders, coating agents, lubricants, lubricating agents, flavoring agents, sweeteners, and solubilizers.

The dosage and the number of doses of the pharmaceutical composition may be appropriately set by those skilled in the art on the basis of factors such as the animal species, health condition, age, and weight of the subject, the administration route, and the employed dosage form, so that an effective amount of a compound of formula (I) is administered to the subject. Those skilled in the art may easily determine the effective amount in a given situation by routine experimentation, using ordinary skill and determination of clinicians. For example, a compound of formula (I) may be administered in the range of about 0.001 to 1000 mg/kg body weight/day, about 0.01 to 300 mg/kg body weight/day, about 0.1 to 100 mg/kg body weight/day, or about 1 to 100 mg/kg body weight/day. The pharmaceutical composition may be administered in a single dose or in multiple doses, or may be chronically administered. When administered in multiple doses or chronically, the pharmaceutical composition may be administered, for example, once to several times a day, e.g., once, twice, or three times a day, daily or every few days, e.g., every one, two, three, or seven days. The duration of administration is not limited and may range from one day to the lifetime of the subject. For example, when administered in multiple doses, the pharmaceutical composition may be administered from one day to several months (e.g., one day to six months, one day to three months, one day to two months, or one day to one month), from one day to several weeks (e.g., one day to three weeks, one day to two weeks, one day to one week), from one day to several days (e.g., one day to three days or one day to two days), or in one day. When administered chronically, the pharmaceutical composition may be administered through the lifetime of the subject, during which the administration may be interrupted temporarily.

A compound of formula (I) may be used alone or in combination with at least one further active ingredient, especially an active ingredient for protecting cartilage or for treating and/or preventing a disease associated with cartilage degeneration. For example, the pharmaceutical composition may contain at least one further active ingredient in addition to a compound of formula (I).

When some ingredients are used in combination, a dosage form containing all the ingredients or a combination of dosage forms containing the ingredients separately may be employed. Multiple ingredients may be simultaneously administered or any ingredient may be administered at a later time point, as long as the ingredients are used for protecting cartilage or for treating and/or preventing a disease associated with cartilage degeneration. Two or more further active ingredients may be used in combination. Examples of the active ingredients suitable for use in combination include, but are not limited to, non-steroidal antiinflammatory agents, steroids, hyaluronic acid, and local anesthetics.

A non-drug therapy may be combined with the administration of a compound of formula (I). Examples of the suitable therapies include ligament reconstruction, arthroplasty, osteosynthesis, and regenerative medicine.

An aspect of the disclosure provides a method of protecting cartilage comprising administering an effective amount of a compound of formula (I) to a subject in need thereof.

An aspect of the disclosure provides a compound of formula (I) for use in protecting cartilage.

An aspect of the disclosure provides use of a compound of formula (I) for protecting cartilage.

An aspect of the disclosure provides use of a compound of formula (I) for manufacturing a pharmaceutical composition for protecting cartilage.

An aspect of the disclosure provides a method of treating and/or preventing a disease associated with cartilage degeneration comprising administering an effective amount of a compound of formula (I) to a subject in need thereof.

An aspect of the disclosure provides a compound of formula (I) for use in treating and/or preventing a disease associated with cartilage degeneration.

An aspect of the disclosure provides use of a compound of formula (I) for treating and/or preventing a disease associated with cartilage degeneration.

An aspect of the disclosure provides use of a compound of formula (I) for manufacturing a pharmaceutical composition for treating and/or preventing a disease associated with cartilage degeneration.

For example, the disclosure provides the following embodiments.
[1] A pharmaceutical composition for protecting cartilage, comprising a compound of formula (I): 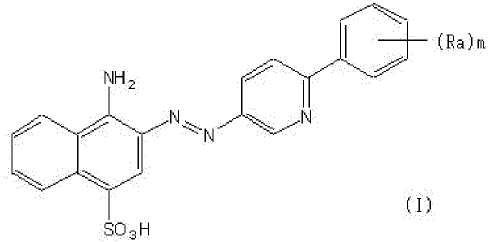 wherein
   Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
   m is an integer selected from 0 to 4,
   or an ester, oxide, pharmaceutically acceptable salt, or solvate thereof.
[2] The pharmaceutical composition according to item 1, which is for protecting a chondrocyte.
[3] The pharmaceutical composition according to item 1 or 2, which is for suppressing death of a chondrocyte.
[4] The pharmaceutical composition according to any one of items 1 to 3, which is for treating and/or preventing a disease associated with cartilage degeneration.
[5] A pharmaceutical composition for treating and/or preventing a disease associated with cartilage degeneration, comprising a compound of formula (I): 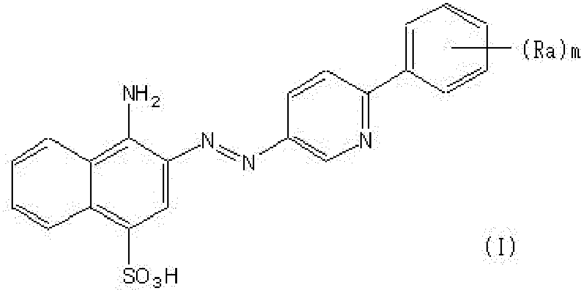 wherein
   Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
   m is an integer selected from 0 to 4,
   or an ester, oxide, pharmaceutically acceptable salt, or solvate thereof.
[6] The pharmaceutical composition according to item 4 or 5, wherein the disease associated with cartilage degeneration is osteoarthritis.
[7] The pharmaceutical composition according to item 6, wherein the osteoarthritis is primary osteoarthritis or secondary osteoarthritis.
[8] The pharmaceutical composition according to item 6 or 7, wherein the osteoarthritis is post-traumatic secondary osteoarthritis.
[9] The pharmaceutical composition according to any one of items 6 to 8, wherein the osteoarthritis is temporomandibular joint osteoarthritis, knee osteoarthritis, hip osteoarthritis, ankle osteoarthritis, shoulder osteoarthritis, elbow osteoarthritis, wrist osteoarthritis, finger osteoarthritis, or spondylosis.
[10] The pharmaceutical composition according to any one of items 1 to 9, wherein each Ra radical is independently selected from the group consisting of halo, hydroxy, alkyl, halo-substituted alkyl, and alkoxy.
[11] The pharmaceutical composition according to any one of items 1 to 10, wherein each Ra radical is independently selected from the group consisting of halo and alkyl.
[12] The pharmaceutical composition according to any one of items 1 to 11, wherein formula (I) has two Ra radicals which are halo and alkyl.
[13] The pharmaceutical composition according to any one of items 1 to 12, wherein the compound of formula (I) is selected from the compounds listed in Table 1.
[14] The pharmaceutical composition according to any one of items 1 to 13, wherein the compound of formula (I) is 4-amino-3-[6-(4-fluoro-2-methylphenyl)pyridine-3-ylazo]naphthalene-1-sulfonic acid.
[15] The pharmaceutical composition according to any one of items 1 to 14, wherein the pharmaceutical composition is administered intraarticularly.
[16] The pharmaceutical composition according to any one of items 1 to 14, wherein the pharmaceutical composition is administered intravenously.

The entire contents of the documents cited herein are incorporated herein by reference.

The following examples are non-limiting and provided only for describing the invention. The embodiments described above are non-limiting and may be modified without deviating from the scope of the invention as defined by the appended claims.

### EXAMPLES

In the examples, the following compound was employed.
KUS121: 4-amino-3-[6-(4-fluoro-2-methylphenyl)pyridine-3-ylazo]naphthalene-1-sulfonic acid sodium salt
KUS121 was prepared by the method disclosed in WO2012/014994.

### Example 1

### Effects of KUS121 in cultured chondrocytes

### Cell culture and differentiation

Cells of mouse teratocarcinoma-derived cell line ATDC5 (J Cell Biol. 1996 Apr;133(2):457-68) were cultured in DMEM/Ham's F12 medium containing 5% FBS. The cells were passaged to a 12-well plate at the density of 3 × 10⁴ cells/well and cultured in the presence of insulin (10 µg/ml) (start of differentiation induction, Day 0). The cells were differentiated into chondrocytes by differentiation induction for 14 to 21 days. The cells were used in the following experiments.

### Tm stress

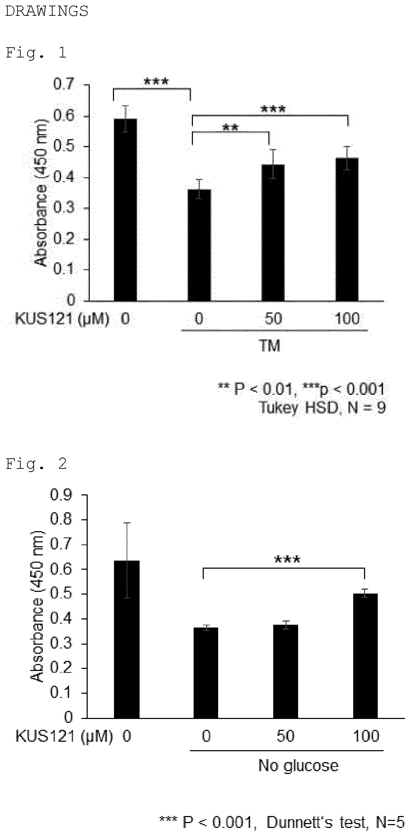

On Day 15 of the differentiation induction, tunicamycin (0.2 µg/mL), which induces ER stress, and KUS121 (50 or 100 µM) were added to the medium and the chondrocytes were cultured for 40 hours. Relative number of viable cells was determined by using 2-(2-methoxy-4-nitrophenyl)-3-(4-nitrophenyl)-5-(2,4-disulfophenyl)-2H-tetrazolium monosodium salt (WST8). The results are shown in Fig. 1. Cell death was significantly suppressed in the presence of KUS121.

### Glucose starvation stress

The chondrocytes were cultured in the medium containing no glucose and containing KUS 121 (50 or 100 µM) for five days from Day 17 of the differentiation induction. Relative number of viable cells was determined by using WST8. The results are shown in Fig. 2. Cell death was significantly suppressed in the presence of 100 µM KUS121.

### TNFα stress

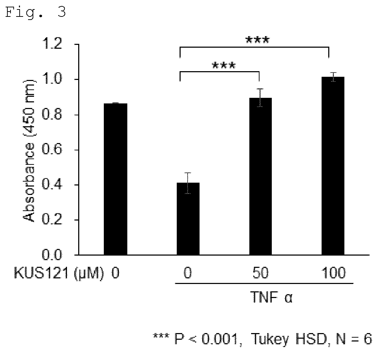

On Day 15 of the differentiation induction, TNFα (10 ng/mL), which induces apoptosis, and KUS121 (50 or 100 µM) were added to the medium and the chondrocytes were cultured for 48 hours. Relative number of viable cells was determined by using WST8. The results are shown in Fig. 3. Cell death was significantly suppressed in the presence of KUS121.

### Measurement of stress markers by Western blotting

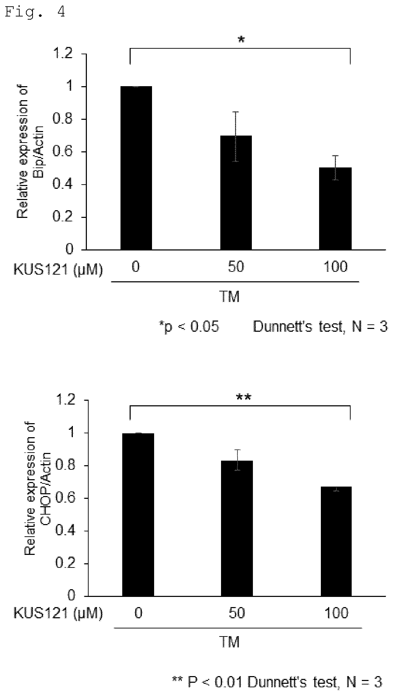

On Day 15 of the differentiation induction, tunicamycin (3 µg/mL) and KUS121 (50 or 100 µM) were added to the medium and the chondrocytes were cultured for 6 hours. The cells were subjected to Western blotting. As primary antibodies, Bip (Cell Signaling #3177S), CHOP (Proteintech #15204-1-AP), and actin (Sigma #A5441) were used. The results are shown in Fig. 4. Expression of Bip and CHOP was suppressed in the presence of KUS121. Bip is an ER stress marker and CHOP induces apoptosis related to ER stress.

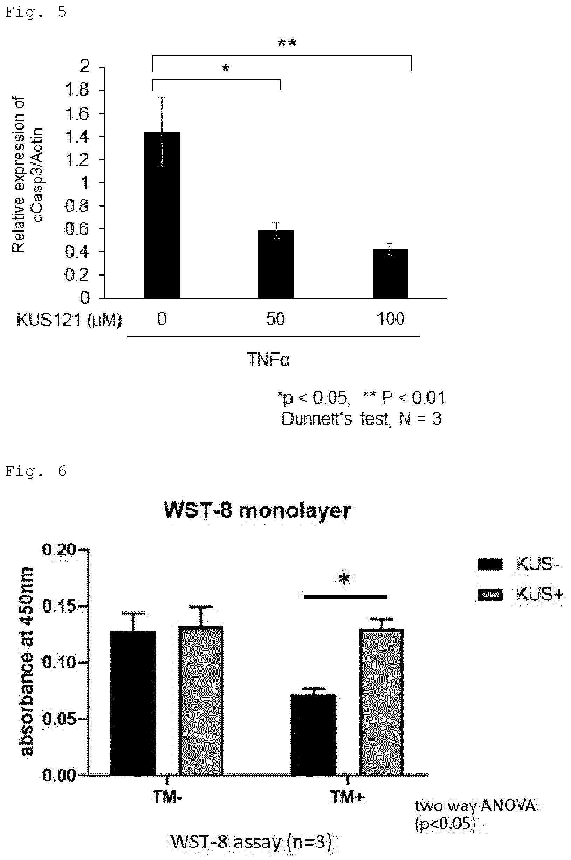

On Day 15 of the differentiation induction, TNFα (20 ng/mL) and KUS121 (50 or 100 µM) were added to the medium and the chondrocytes were cultured for 48 hours. The cells were subjected to Western blotting. As primary antibodies, cCasp3 (Cell Signaling #9661S) and actin (Sigma #A5441) were used. The results are shown in Fig. 5. Expression of Casp3, an apoptosis marker, was suppressed in the presence of KUS121.

### Example 2

### Effects of KUS121 in monolayer culture of human chondrocytes

Chondrocytes were collected from a knee joint removed from a patient in knee replacement surgery. The cells were cultured in DMEM-F12 (Gibco, 11330-032) on a 24-well plate to 70-80% confluence and cultured in the medium without FBS for further 24 hours. The cells were then cultured in the medium containing tunicamycin (3 µg/mL) and KUS121 (100 µM) for 24 hours. The medium was replaced with the medium containing 0.5% FBS and the cells were cultured for 48 hours. For counting viable cells, CCK-8 (DOJINDO LABORATORIES) was added, which is a kit for cell counting comprising WST8 as a chromogenic substrate. The cells were incubated for 15 minutes at 37°C and the absorbances (450 nm, 570 nm) were determined. The results are shown in Fig. 6. Viable cells were decreased by adding tunicamycin, but not decreased in the presence of KUS121.

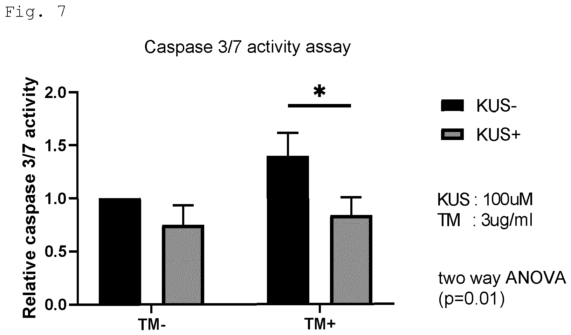

Human chondrocytes were cultured to 70-80% confluence. Tunicamycin (3 µg/mL) and KUS121 (100 µM) were added to the medium and the cells were cultured for further 6 hours. Casp3/7 Glo assay (Promega) was used in accordance with the manufacture's instructions and caspase 3/7 activity was determined by luminometer. The results are shown in Fig. 7. The caspase activity was increased by adding tunicamycin, but not increased in the presence of KUS121.

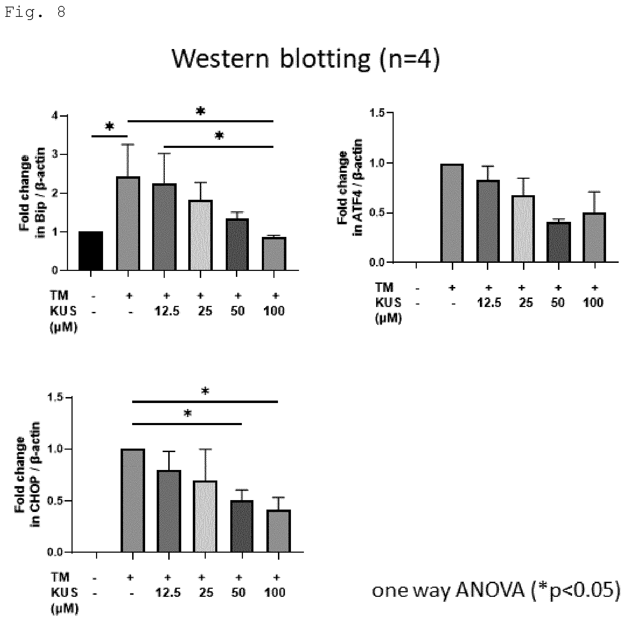

Human chondrocytes were cultured to 70-80% confluence. Tunicamycin (3 µg/mL) and KUS121 (12.5, 25, 50, or 100 µM) were added to the medium and the cells were cultured for further 8 hours. Proteins were collected from the cells and subjected to Western blotting. As primary antibodies, Bip (Cell Signaling Technology, #3177), ATF4 (Cell Signaling Technology, #11815), CHOP (Cell Signaling Technology, #2895), and β-actin (Cell Signaling Technology, #5125) were used. The results are shown in Fig. 8. In the absence of tunicamycin expressions of ATF4 and CHOP were observed, but the expression levels were quite low. In the presence of tunicamycin KUS121 suppressed expression of Bip, ATF4, and CHOP in a dose-dependent manner. The results indicate KUS121 suppresses apoptosis related to ER stress.

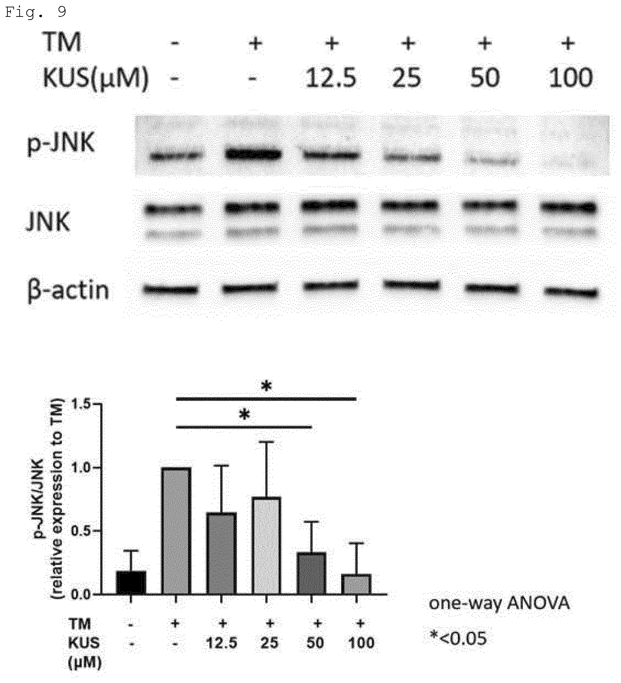

Human chondrocytes were cultured to 70-80% confluence. Tunicamycin (3 µg/mL) and KUS121 (12.5, 25, 50, or 100 µM) were added to the medium and the cells were cultured for further 8 hours. Proteins were collected from the cells and subjected to Western blotting. As primary antibodies, p-JNK (Cell Signaling Technology, #4668), JNK (Cell Signaling Technology, #9252), and β-actin (Cell Signaling Technology, #5125) were used. The results are shown in Fig. 9. KUS121 suppressed expression of phosphorylated JNK (p-JNK), a MAP kinase, in a dose-dependent manner.

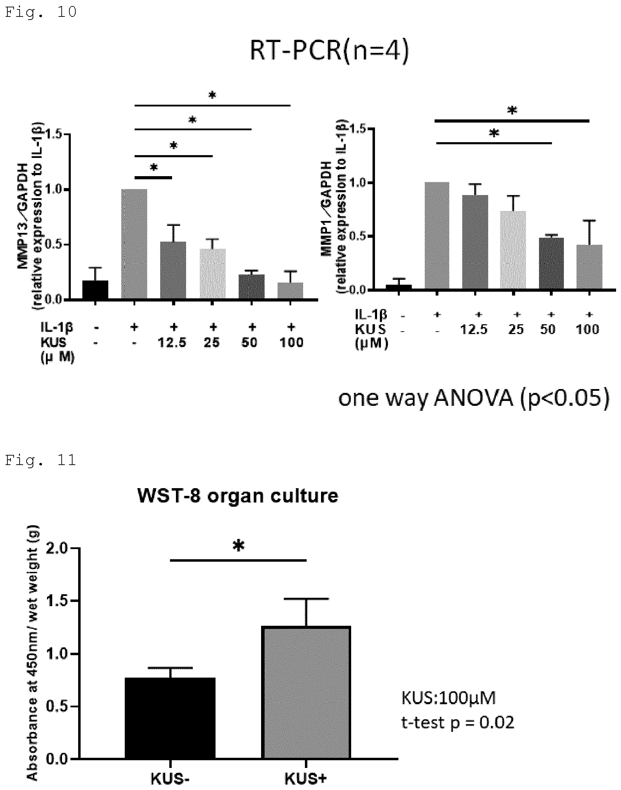

Human chondrocytes were cultured on a 24-well plate to 70-80% confluence and cultured in the medium without FBS for 24 hours. The cells were then cultured in the medium containing IL-1β (2 ng/mL) and KUS121 (12.5, 25, 50, or 100 µM) for 24 hours. Messenger RNAs were extracted from the cells and cDNAs were generated by reverse transcription. Gene expression levels of MMP13 and MMP1 were determined by real-time PCR. The results are shown in Fig. 10. KUS121 suppressed expression of MMP13 and MMP1, which are collagenases, in a dose-dependent manner.

### Example 3

### Effects of KUS121 in organ culture of human cartilage

From a knee joint removed from a patient in knee replacement surgery cartilage of femorotibial joint (posterolateral femoral condyle) was collected by using a biopsy punch having a diameter of 7 mm. The cartilage was cultured in the medium without FBS for 48 hours and then in the medium containing KUS121 (100 µM) for 72 hours. Number of viable cells was determined by washing the cartilage with PBS, adding CCK-8, incubating the cartilage for 30 minutes at 37°C, and determining the absorbances (450 nm, 570 nm). The results are shown in Fig. 11. Number of viable cells was larger in the presence of KUS121. It is suggested that in this experiment the chondrocytes of the patient had been burdened by various stresses until the collection and KUS121 reduced the stresses.

### Example 4

### Effects of KUS121 in knee osteoarthritis model

It is known that intraarticularly administered monoiodoacetate (MIA) damages chondrocytes and suppresses secretin of collagen and proteoglycan to induce osteoarthritis (M. Udo et al., Osteoarthritis Cartilage. 2016, 24:1284-91). Knee osteoarthritis model rats were generated by administering 0.5 mg of monoiodoacetate (MIA) in 25 µl of saline solution into knee joint cavities of both hind limbs of healthy rats. Prior to the MIA administration, 50 mg/Kg/day of KUS121 was administered intraperitoneally for four days. After the MIA administration, the same amount of KUS121 was administered intraperitoneally five days per week.

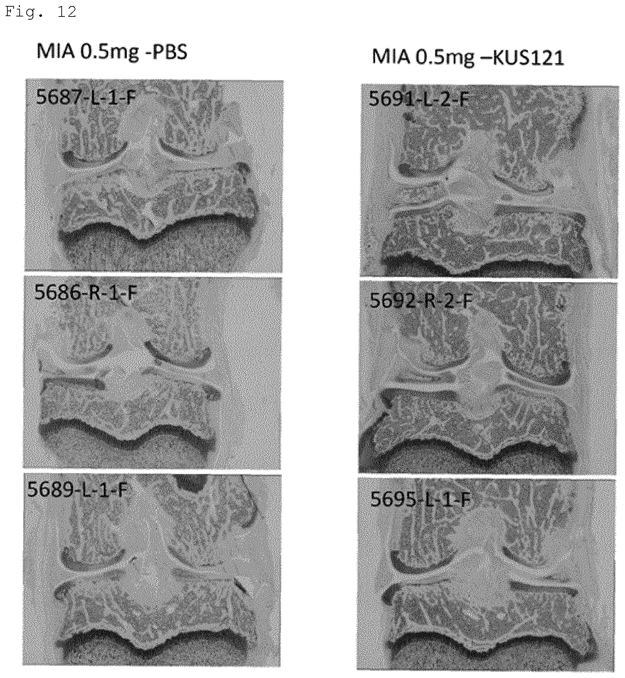

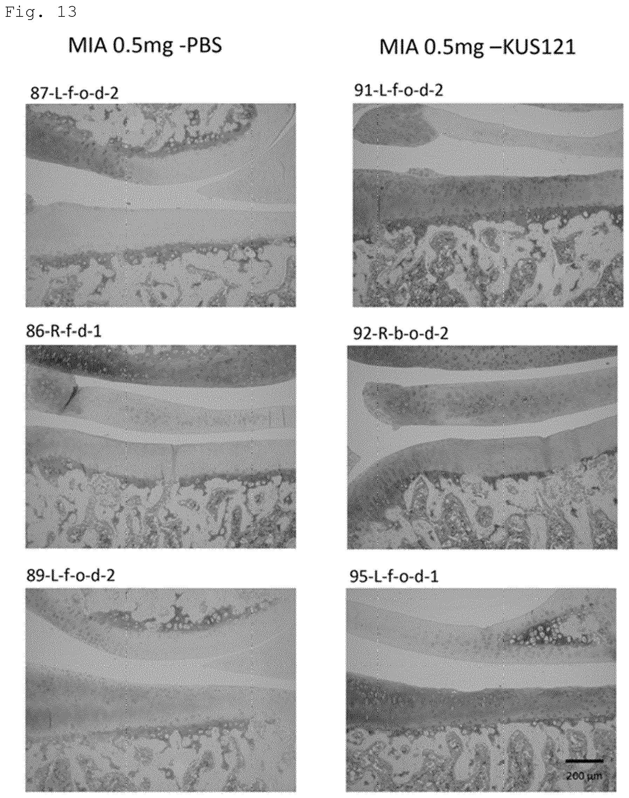

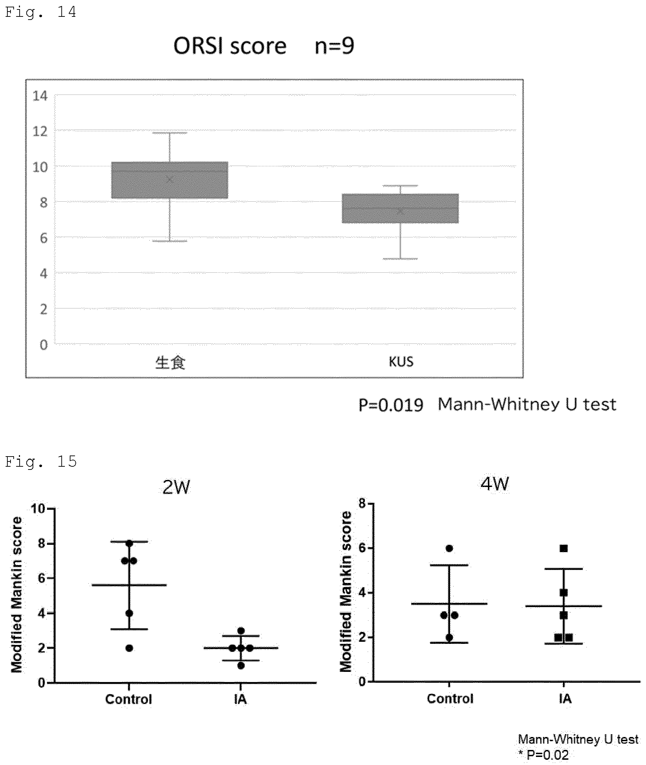

Two weeks after the MIA administration the knee cartilages of the rats were resected, fixed with 10% neutral buffered formalin for about one week, demineralized with Morse's solution (for two weeks) or EDTA (for one month), cleaved along the medial collateral ligament, and embedded in paraffin with the cut surface facing up. Sections were cut every 100-200 µm with a thickness of 3-6 µm. The sections were stained with Safranin-O, which stains chondrocytes, and observed with a fluorescence microscope (KEYENCE BZ9000). Representative results are shown in Fig. 12. Fig. 13 shows magnified images of the tibial joints. The staining, which indicates chondrocytes, was darker in the KUS121-treated group than in the control group (PBS). In addition, three blinded evaluators evaluated ORSI scores for medial femur, lateral femur, medial tibia, and lateral tibia on the basis of the results of the Safranin-O staining. ORSI scores are used for grading knee osteoarthritis (Osteoarthritis cartilage histopathology; Osteoarthritis Cartilage. 2006 Jan;14(1):13-29). The results are shown in Fig. 14. The score was lower in the KUS121-treated group than in the control group. The results indicate the cartilage damage was suppressed by administering KUS121.

### Example 5

### Effects of KUS121 in traumatic cartilage damage model

It is known that a mouse that has been given periodic compression to a knee joint can be used as a model of post-traumatic cartilage damage (Wu P, Sandell L, et al. Arthritis Rheumatol. 2014;66(5):1256-65). In a similar manner, a rat post-traumatic cartilage damage model was generated. Specifically, a knee of a hind limb was deeply flexed and fixed on the table of Autograph (Shimadzu Corporation) with the patella facing down. A vertical load of 5 N was applied downward from the heel, then 240 loads of 20 N at the maximum were applied with 5-second intervals. After eight weeks the posterolateral femoral condyle was collected and stained with Safranin-O. Decreased staining, which suggests cartilage damage, was observed. This model was used for the following experiments.

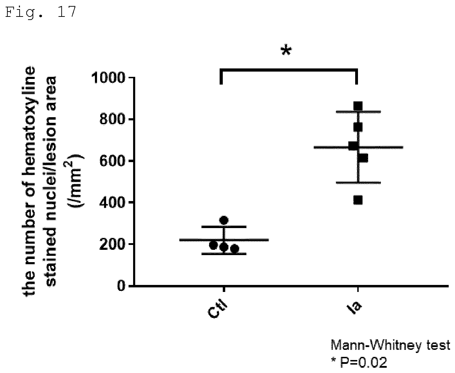

Knee joints of 12-week old male Wister rats (five rats per group) were given periodic compression by the method described above. KUS121 (20 mg/ml) in 5% glucose solution (100 µl) was intraarticularly administered to rats in KUS 121 group immediately before the operation and at Day 1, 2, and 3 after the operation. To rats in the control group 5% glucose solution (100 µl) was administered. The rats were sacrificed two or four weeks after the operation. Damages of posterolateral femoral condyle were histologically evaluated. Fig. 15 shows Modified Mankin scores, which are indicators of articular cartilage degeneration, evaluated two and four weeks after the operation. Two weeks after the operation, the score of the treated group (IA) was significantly lower than that of the control group, indicating a tendency of suppressed apoptosis. Four weeks after the operation, no significant difference was observed. Four weeks after the operation, the volume of cartilage damage was significantly reduced in the treated group (Fig. 16). Four weeks after the operation, the number of chondrocytes was maintained at a significantly higher level in the treated group (Fig. 17).

### INDUSTRIAL APPLICABILITY

The disclosure provides the method for protecting cartilage and thus may be used in the field of medicine, for example, for treating and/or preventing a disease associated with cartilage degeneration such as osteoarthritis.

## Claims

1. A pharmaceutical composition for protecting cartilage, comprising a compound of formula (I): 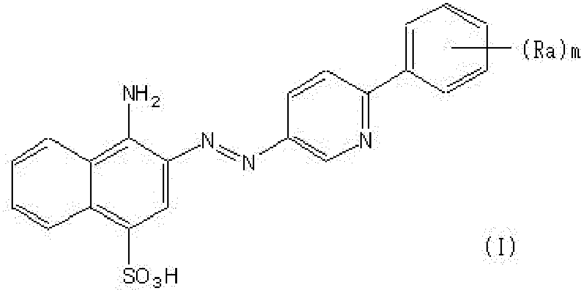 wherein
Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
m is an integer selected from 0 to 4,
or an ester, oxide, pharmaceutically acceptable salt, or solvate thereof.

2. The pharmaceutical composition according to claim 1, which is for protecting a chondrocyte.

3. The pharmaceutical composition according to claim 1 or 2, which is for treating and/or preventing a disease associated with cartilage degeneration.

4. The pharmaceutical composition according to claim 3, wherein the disease associated with cartilage degeneration is osteoarthritis.

5. A pharmaceutical composition for treating and/or preventing a disease associated with cartilage degeneration, comprising a compound of formula (I): 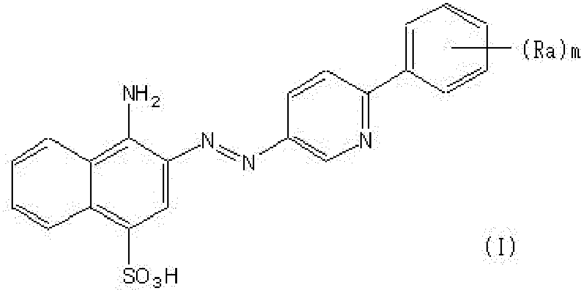 wherein
Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
m is an integer selected from 0 to 4,
or an ester, oxide, pharmaceutically acceptable salt, or solvate thereof.

6. The pharmaceutical composition according to claim 5, wherein the disease associated with cartilage degeneration is osteoarthritis.

7. The pharmaceutical composition according to any one of claims 1 to 6, wherein each Ra radical is independently selected from the group consisting of halo, hydroxy, alkyl, halo-substituted alkyl, and alkoxy.

8. The pharmaceutical composition according to any one of claims 1 to 6, wherein the compound of formula (I) is selected from the group consisting of
4-amino-3-(6-phenylpyridine-3-ylazo)naphthalene-1-sulfonic acid;
4-amino-3-(6-p-tolylpyridine-3-ylazo)naphthalene-1-sulfonic acid;
4-amino-3-(6-m-tolylpyridine-3-ylazo)naphthalene-1-sulfonic acid;
4-amino-3-(6-o-tolylpyridine-3-ylazo)naphthalene-1-sulfonic acid;
4-amino-3-(6-biphenyl-2-ylpyridine-3-ylazo)naphthalene-1-sulfonic acid;
3-[6-(2-acetylphenyl)pyridine-3-ylazo]-4-aminonaphthalene-1-sulfonic acid;
3-[6-(3-acetylphenyl)pyridine-3-ylazo]-4-aminonaphthalene-1-sulfonic acid;
3-[6-(4-acetylphenyl)pyridine-3-ylazo]-4-aminonaphthalenesulfonic acid;
4-amino-3-[6-(2,4-dichlorophenyl)pyridine-3-ylazo]naphthalene-1-sulfonic acid;
4-amino-3-[6-(2-trifluoromethylphenyl)pyridine-3-ylazo]naphthalene-1-sulfonic acid;
4-amino-3-[6-(4-trifluoromethylphenyl)pyridine-3-ylazo]naphthalene-1-sulfonic acid;
4-amino-3-[6-(2-chlorophenyl)pyridine-3-ylazo]naphthalene-1-sulfonic acid;
4-amino-3-[6-(3-chlorophenyl)pyridine-3-ylazo]naphthalene-1-sulfonic acid;
4-amino-3-[6-(4-chlorophenyl)pyridine-3-ylazo]naphthalene-1-sulfonic acid;
4-amino-3-[6-(2-methoxyphenyl)pyridine-3-ylazo]naphthalene-1-sulfonic acid;
4-amino-3-[6-(4-methoxyphenyl)pyridine-3-ylazolnaphthalene-1-sulfonic acid; 4-amino-3-[6-(2-isopropoxyphenyl)pyridine-3-ylazo]naphthalene-1-sulfonic acid; 4-amino-3-[6-(4-isopropoxyphenyl)pyridine-3-ylazo]naphthalene-1-sulfonic acid; 4-amino-3-[6-(2-phenoxyphenyl)pyridine-3-ylazo]naphthalene-1-sulfonic acid; 4-amino-3-[6-(3-methoxyphenyl)pyridine-3-ylazo]naphthalene-1-sulfonic acid; 4-amino-3-[6-(2,3-dimethylphenyl)pyridine-3-ylazo]naphthalene-1-sulfonic acid; 4-amino-3-[6-(2,5-dimethylphenyl)pyridine-3-ylazo]naphthalene-1-sulfonic acid; 4-amino-3-[6-(3,5-dimethylphenyl)pyridine-3-ylazo]naphthalene-1-sulfonic acid; 4-amino-3-[6-(3-trifluoromethylphenyl)pyridine-3-ylazo]naphthalene-1-sulfonic acid; 4-{4-[5-(1-amino-4-sulfonaphthalene-2-ylazo)pyridine-2-yl]phenyl}-4-oxobutyric acid; 4-amino-3-(6-biphenyl-3-ylpyridine-3-ylazo)naphthalene-1-sulfonic acid; 4-amino-3-[6-(3-cyanophenyl)pyridine-3-ylazo]naphthalene-1-sulfonic acid; 4-amino-3-[6-(4-cyanophenyl)pyridine-3-ylazo]naphthalene-1-sulfonic acid; 4-amino-3-[6-(3,5-bistrifluoromethylphenyl)pyridine-3-ylazo]naphthalenesulfonic acid; 4-amino-3-[6-(4-benzoylphenyl)pyridine-3-ylazo]naphthalene-1-sulfonic acid; 4-amino-3-[6-(2-propoxyphenyl)pyridine-3-ylazo]naphthalene-1-sulfonic acid; 4-amino-3-[6-(4-fluoro-2-methylphenyl)pyridine-3-ylazo]naphthalene-1-sulfonic acid;
4-amino-3-[6-(5-fluoro-2-propoxyphenyl)pyridine-3-ylazo]naphthalene-1-sulfonic acid;
4-amino-3-[6-(2-fluoro-6-propoxyphenyl)pyridine-3-ylazo]naphthalene-1-sulfonic acid;
4-amino-3-[6-(4-fluoro-2-propoxyphenyl)pyridine-3-ylazo]naphthalene-1-sulfonic acid;
4-amino-3-[6-(5-fluoro-2-methylphenyl)pyridine-3-ylazo]naphthalene-1-sulfonic acid;
4-amino-3-[6-(2-fluoro-5-methylphenyl)pyridine-3-ylazo]naphthalene-1-sulfonic acid;
4-amino-3-[6-(2-butoxyphenyl)pyridine-3-ylazo]naphthalene-1-sulfonic acid;
4-amino-3-[6-(2-hexyloxyphenyl)pyridine-3-ylazo]naphthalene-1-sulfonic acid;
4-amino-3-[6-(4-butylphenyl)pyridine-3-ylazo]naphthalene-1-sulfonic acid;
4-amino-3-[6-(2-hydroxyphenyl)pyridine-3-ylazo]naphthalene-1-sulfonic acid;
4-amino-3-{6-[2-(6-hydroxyhexyloxy)phenyl]pyridine-3-ylazo}naphthalene-1-sulfonic acid;
4-{2-[5-(1-amino-4-sulfonaphthalene-2-ylazo)pyridine-2-yl]phenoxy} butyric acid;
4-amino-3-{6-[2-(3-hydroxypropoxy)phenyl]pyridine-3-ylazo}naphthalene-1-sulfonic acid;
4-amino-3-[6-(2-isobutoxyphenyl)pyridine-3-ylazo]naphthalene-1-sulfonic acid;
4-amino-3-[6-(5-chloro-2-hydroxyphenyl)pyridine-3-ylazo]naphthalene-1-sulfonic acid;
4-amino-3-[6-(4-methylbiphenyl-2-yl)pyridine-3-ylazo]naphthalene-1-sulfonic acid;
4-amino-3-[6-(4'-chloro-4-methylbiphenyl-2-yl)pyridine-3-ylazo]naphthalene-1-sulfonic acid;
4-amino-3-[6-(4,3',5'-trimethylbiphenyl-2-yl)pyridine-3-ylazo]naphthalene-1-sulfonic acid;
4-amino-3-[6-(3'-chloro-4-methylbiphenyl-2-yl)pyridine-3-ylazo]naphthalene-1-sulfonic acid;
4-amino-3-[6-(2,6-dimethylphenyl)pyridine-3-ylazo]naphthalene-1-sulfonic acid;
4-amino-3-[6-(3-formyl-2-isopropoxy-5-methylphenyl)pyridine-3-ylazo]naphthalene-1-sulfonic acid; and
4-amino-3-[6-(3-formyl-2-butoxy-5-methylphenyl)pyridine-3-ylazo]naphthalene-1-sulfonic acid.

9. The pharmaceutical composition according to any one of claims 1 to 8, wherein the compound of formula (I) is 4-amino-3-[6-(4-fluoro-2-methylphenyl)pyridine-3-ylazo]naphthalene-1-sulfonic acid.

10. The pharmaceutical composition according to any one of claims 1 to 9, wherein the pharmaceutical composition is administered intraarticularly.

11. The pharmaceutical composition according to any one of claims 1 to 9, wherein the pharmaceutical composition is administered intravenously.
